Europäisches Patentamt

(19) European Patent Office

Office européen des brevets

(11) Publication number: **0 192 332**
**A1**

(12) # EUROPEAN PATENT APPLICATION

(21) Application number: **86300323.2**

(22) Date of filing: **17.01.86**

(51) Int. Cl.⁴: **C 12 P 19/26**
**C 12 P 21/00**

(30) Priority: **22.01.85 US 692951**

(43) Date of publication of application:
**27.08.86 Bulletin 86/35**

(84) Designated Contracting States:
**CH DE FR GB IT LI NL**

(71) Applicant: **MERCK & CO. INC.**
**126, East Lincoln Avenue P.O. Box 2000**
**Rahway New Jersey 07065(US)**

(72) Inventor: **Richey, Danny D.**
**3763 Cascade Court**
**San Diego California 92122(US)**

(72) Inventor: **Dohner, John F.**
**6819 Bonnie View Drive**
**San Diego California 92119(US)**

(72) Inventor: **Lewis, Jerry G.**
**1353 Vue de Ville**
**San Diego California 92109(US)**

(74) Representative: **Crampton, Keith John Allen et al,**
**D YOUNG & CO 10 Staple Inn**
**London WC1V 7RD(GB)**

(54) **Biosynthetic polysaccharide and process.**

(57) A novel biosynthetic polysaccharide has a Brookfield viscosity, in 0.25% aqueous solution, of at least about 2000 mPa.s. It is prepared by aerobic fermentation of the ATCC 31961 organism in a medium containing a carbon source, deionized water and hydrolysed soy protein.

1

# BIOSYNTHETIC POLYSACCHARIDE AND PROCESS

The invention is concerned with a high viscosity biosynthetic polysaccharide and its preparation by aerobic fermentation.

U.S. Patent Specification US-A-4,401,760 describes the general preparation of a polysaccharide by aerobic fermentation of the Alcaligenes microorganism ATCC 31961.

A specific process has been developed by which the polysaccharide prepared by aerobic fermentation of the said microorganism has a viscosity, in 0.25% aqueous solution, of over about 2000 mPa.s (Brookfield 3 rpm, No. 2 spindle).

In accordance with one embodiment of the invention, a biosynthetic polysaccharide having a Brookfield (Model LVT or LVF) viscosity, in 0.25 weight % aqueous solution, of at least about 2000 mPa.s is prepared by aerobic fermentation of the Alcaligenes organism ATCC 31961 in a deionized aqueous nutrient medium containing a carbon source and a hydrolysed soy protein.

Preparation of the culture seed and general fermentation conditions, except the nutrient medium, are substantially the same as those disclosed in the said US-A-4,401,760.

A characterizing feature of the present invention is the nutrient medium composition. The composition contains the following major essential ingredients: a carbon source, a hydrolysed soy protein and deionized water.

Useful carbon sources are water soluble materials such as corn syrup and glucose. Useful hydrolysed soy protein includes enzyme hydrolysed soybean meal and preferably a papain hydrolysed soybean meal extract.

Viscosity as used herein is that measured using a Brookfield Model LVT or LVF viscometer, No. 2 spindle at 3 rpm.

Another embodiment of the invention is the polysaccharide having the high viscosity (over about 2000) and obtainable by the process described herein.

Other identifying characteristics of the polysaccharide will be provided below.

A further embodiment is the treatment of
high viscosity S-194 of this invention with
dialdehyde of from 2 to 8 carbon atoms, preferably
glyoxal, to form a complex which is readily
dispersible in water. The complex is formed by
reacting 0.02% to 5% by weight dialdehyde based on
dry weight of S-194. The reaction takes place at
about 50°-150°C, preferably 60°-120°C, in mildly
acidic pH, about 5.2-6.0.

Any of various means can be used to effect
the reaction. For example, dialdehyde may be added
to S-194 fermentate prior to or during precipitation. Alternatively, dry S-194 powder may be
reconstituted with water or in a water/alcohol
mixture and then reacted with dialdehyde. Instead of
fully hydrating the S-194, a lesser amount of water
can be used to produce a slurry wherein the reaction
occurs. The reaction also can be effected by dry
mixing, i.e., using dry ingredients. Using this
method S-194 and dialdehyde are intimately intermixed
as in a tumbler or by using a mortar and pestle.
While tumbling the S-194, dialdehyde can also be
sprayed thereon. These and similar processes are
within the scope of this embodiment.

A further embodiment is the dialdehyde/S-194
complex, comprising 0.02 to 5% dialdehyde and high
viscosity S-194.

The uses of this polysaccharide include
those described in U.S. 4,401,760 as well as use in
preparing coal-in-water slurries containing 60% or
more ground coal.

3

Following are examples illustrating preparation of polysaccharides, including those of the present invention. Mesh sizes are U.S. standards, unless otherwise indicated.

EXAMPLE 1

The fermentation procedure described in U.S. 4,401,760 was used to prepare the polysaccharides. The fermentor medium used was that, substantially as set out below, and disclosed in U.S. 4,401,760, Column 5 lines 10-17.

Fermentor Medium A

Tap Water

3.0% Glucose

0.05% $K_2HPO_4$

0.20% PROMOSOY 100 *

0.01% $MgSO_4.7H_2O$

0.09% $NH_4NO_3$

0.01-0.05% Antifoam

* - Soy protein concentrate obtained from Central Soya

All fermentations reported herein were run to substantially complete depletion of the carbon source.

The fermentation was carried out in commercial fermentors. Following is a tabulation of a number of fermentation batches and viscosity of the polysaccharide products, in 0.25% aqueous solution, using a Brookfield viscometer (Model LVT or LVF), No. 2 spindle, at 3 rpm.

4

| Batch | 0.25% Viscosity |
|-------|-----------------|
| 1 | 2000 |
| 2 | 1600 |
| 3 | 1900 |
| 4 | 1250 |
| 5 | 1950 |
| 6 | 340 |
| 7 | 1050 |
| 8 | 1300 |
| 9 | 1450 |
| 10 * | 1500 |
| 11 * | 1550 |
| Avg. | 1444 |

* - Corn syrup was substituted for glucose in the fermentation medium.

## EXAMPLE 2

S-194 type polysaccharides were prepared using substantially the same fermentation procedure as in Example 1 but substituting corn syrup for glucose, deionized (D.I.) water for tap water and HY SOY T for PROMOSOY in the fermentor medium A formula. HY SOY T is a papain digested soybean meal extract obtained from Sheffield Products, a Division of Kraft, Inc.

Following is a tabulation of data for S-194 batches so prepared.

5

|  | 0.25% |
| Batch | Viscosity |
| A | 2310 |
| B | 2210 |
| C | 2240 |
| D | 2770 |
| E | 3160 |
| F | 2600 |
| G | 2470 |
| H | 2780 |
| I | 2620 |
| J | 2150 |
| K | 1380 * |
| L | 2560 |
| M | 2490 |
| N | 2790 |
| O | 2770 |
| P | 2100 |
| Avg. | 2462 |

* - Suspected contamination with tap water or culture
was weak.

The data in the tables above clearly
demonstrate that the use of a HY SOY and D.I. water
in the fermentor medium (Example 2) allows
preparation of a S-194 type polysaccharide having a
0.25% aqueous solution Brookfield viscosity of
consistently over about 2000.

One advantage of the present high viscosity
( 2000 cP) polysaccharide over the lower viscosity
polysaccharide, e.g. of Example 1, is that it is a
more efficient suspending or stabilizing agent in

0192332

6

aqueous systems e.g. in coal-in-water suspensions and the like. Thus, less of the high viscosity polysaccharide is required to prepare a suspension, resulting in a cost saving.

## EXAMPLE 3

### Dialdehyde Treated S-194

Two S-194 fermentation beers using D.I. water and 6% corn syrup were prepared, containing 2.3% and 1.35% gum. Samples were treated with various amounts of glyoxal (40% solution) and the products tested for dispersibility according to the test method. The data are shown in Table 3-1.

The following procedures were used. The pH was adjusted to 5.2 or 6.0. Following glyoxal addition, the beer was pasteurized to 70°C for 4-5 minutes and then cooled to 40°C. The S-194 was then precipitated with 3 volumes of isopropanol azeotrope (87.7% IPA in water). The precipitated gum was dried, milled through a 40 mesh screen, and tested for dispersibility.

### TABLE 3-1

|  | 2.3% FERMENTATE | | | | 1.35% FERMENTATE | | | | |
|---|---|---|---|---|---|---|---|---|---|
| pH | 6.1 | 5.2 | 5.2 | 5.2 | 6.0 | 6.0 | 6.0 | 6.0 | 6.0 |
| wt of 40% gly/500g beer | 0 | 0 | 0.75 | 1.13 | 0 | 0 | 0.21 | 0.34 | 0.41 |
| Dispersibility | Poor | Poor | Good | Good | Poor | Poor | Poor | Fair | Good |

7

## DISPERSIBILITY
## TEST METHOD

1. 2 g of 40 mesh product is rapidly added to the side of the vortex caused by stirring at 800 rpm with a standard laboratory flat-bladed propeller 198 ml of STW* in a 400 ml beaker.

2. After 30 seconds of stirring, the beaker is removed and the contents poured through a 20 mesh Tyler screen or equivalent.

3. The product is judged dispersible if there are no product lumps on the screen.

* D.I. water containing 1000 ppm NaCl and 147 ppm $CaCl_2.2H_2O$.

0192332

## CLAIMS

1.  A process for preparing a biosynthetic polysaccharide having a Brookfield viscosity, in 0.25% aqueous solution, of at least about 2000 mPa.s. that comprises aerobic fermentation of the <u>Alcaligenes</u> organism ATCC 31961, characterized in that the fermentation is carried out in a deionized aqueous nutrient medium containing a carbon source and hydrolysed soy protein.

2.  A process as claimed in Claim 1 In which the medium additionally contains $K_2HPO_4$, $NH_4NO_3$, $MgSO_4.7H_2O$, $MnCl_2$ and an antifoam agent.

3.  A process as claimed in Claim 1 or 2 in which the protein is a papain hydrolysed soybean meal extract.

4.  A process as claimed in Claim 1 in which the medium contains up to 3% corn syrup, 0.05 to 0.5% $K_2HPO_4$, up to 0.09% $NH_4NO_3$, up to 1 ppm $MgSO_4.7H_2O$, up to 0.2% HY-SOY and up to 0.04% Mazu.

5.  A process as claimed in Claim 4 in which the medium also contains a trace of added $MnCl_2$.

6.  A biosynthetic polysaccharide obtainable by a process as claimed in any one of Claims 1 to 5.

7.  A biosynthetic polysaccharide having a Brookfield viscosity, in 0.25% aqueous solution, of about 2000 mPa.s and higher.

8.  A process for preparing an S-194/dialdehyde complex that comprises mixing 0.02-5% by weight of a dialdehyde with S-194 at 50°-150°C at a mildly acidic pH.

9.  A process as claimed in Claim 8 in which the dialdehyde is glyoxal.

10.  A process as claimed in Claim 8 or 9 in which the dialdehyde is mixed with the S-194 in solution.

11. A process as claimed in Claim 10 in which the solution is of S-194 fermentate.

12. A process as claimed in Claim 8 or 9 in which the dialdehyde is dry mixed with the S-194.

13. A process as claimed in Claim 8 or 9 in which the dialdehyde is mixed with the S-194 in an alcohol/water mixture.

European Patent
Office

**EUROPEAN SEARCH REPORT**

**0192332**
Application number

EP 86 30 0323

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| D,A | EP-A-0 077 680 (MERCK & CO.)<br><br>* Page 7, line 30 *<br><br>--- | | C 12 P 21/00<br>C 12 P 19/26<br>C 08 B 37/00 |
| A | US-A-3 933 788 (KANG et al.)<br><br>* Column 8, lines 53-65 *<br><br>--- | | |
| A | CHEMICAL ABSTRACTS, vol. 89, 1978, page 404, no. 103458x, Columbus, Ohio, US; M. YAMAGUCHI et al.: "Microbial production of polysaccharide. II. Production of viscous polysaccharide from ethylene glycol. (Sugar components and property of polysaccharide)", & BISEIBUTSU KOGYO GIJUTSU KENKYUSHO KENKYU HOKOKU 1977, 49, 103-14<br><br>----- | | |

**TECHNICAL FIELDS SEARCHED (Int. Cl.4)**

C 12 P
C 08 B

*The present search report has been drawn up for all claims*

| Place of search | Date of completion of the search | Examiner |
|---|---|---|
| THE HAGUE | 25-04-1986 | LENSEN H.W.M. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO Form 1503 03 82